# EUROPEAN PATENT SPECIFICATION

(11) **EP 2 117 485 B1**
(45) Date of publication and mention of the grant of the patent: **18.09.2013**
(21) Application number: 08724870.4
(22) Date of filing: 28.01.2008
(51) Int. Cl.: A61F 7/12

(54) **APPARATUS FOR COOLING THE BRAIN USING A GAS**
VORRICHTUNG ZUR KÜHLUNG DES HIRNS MITHILFE VON GAS
APPAREIL DE REFROIDISSEMENT DU CERVEAU AU MOYEN D'UN GAZ

(30) Priority: 26.01.2007 US 897745 P
(43) Date of publication of application: 18.11.2009
(73) Proprietor: Cook Medical Technologies LLC, Bloomington, IN 47404 (US)
(72) Inventor: JOERGENSEN, Alex, DK-2200 Kobenhavn N.- (DK)
(74) Representative: Garratt, Peter Douglas
(86) International application number: PCT/US2008/001090
(87) International publication number: WO 2008/094505

(56) References cited:
- WO-A-2006/124702
- US-A- 5 832 930

## Description

### Technical Field

This invention relates to an apparatus for cooling the brain.

### Background of the Invention

Lowering body temperature is thought to lessen the damaging effects of stroke.

There is a need in the art for an improved brain cooling apparatus which is simple and fast to use in emergency when a patient is in danger of suffering brain damage, for example, following a stroke. It is also desirable to provide a brain cooling apparatus which can be reliably and speedily put into use without significant difficulty by paramedics or nurses in a First Aid situation without requiring intervention by a physician or surgeon.

In addition, there is a need for an improved method of effecting brain cooling in a patient who has suffered a stroke or is otherwise in danger of undergoing brain damage unless speedy medical intervention is applied. In particular, there is a need for a method of effecting controlled cooling of the brain which can be put into operation simply and without the need for complex apparatus or for undertaking difficult medical procedures.

Reference is directed WO 2006124702 which discloses a method for cerebral and systemic cooling by providing a nebulized liquid delivered as a mist or a spray via the nasal and/or oral cavities of a patient. Cooling assemblies are also disclosed that include flexible balloon assemblies that are inserted to various locations in a patient's body. The flexible balloons are then infused with a liquid. The flexible balloon assemblies can be inserted into the nasal cavity, oral cavity, throat, stomach, and other locations to effect cerebral cooling.

### Summary of the Invention

The invention provides an apparatus for cooling the brain comprising: a pernasal intubation tube having a proximal portion, a lumen extending from the proximal portion, a distal end for insertion through one of the nostrils of a patient requiring brain cooling, an inflatable occluder provided on a distal portion of the tube and in communication with the lumen for inflation by fluid supplied through the lumen thereby to occlude the nasopharynx of the patient behind the soft palate of the patient and to prevent passage of liquid between the nasal cavities and the mouth of the patient; a pressure device for application to the exterior of the nose of the patient so as to apply pressure to the angular vein of the patient thereby to increase venous blood flow to the patient's brain; and a coolant gas supply for supplying coolant gas to at least one of the nasal cavities of the patient.

### Brief Description of the Drawings

In the accompanying semi-diagrammatic drawings:
Figure 1 is a vertical section through a patient's left nostril showing a pernasal intubation tube provided in accordance with the invention in position with its soft cuff inflated and with a nasal clip attached to the patient's nose;
Figure 2 is a cross section through the right nostril of the patient of Figure 1 with the pernasal intubation tube shown in dotted lines;
Figure 3A is a cross section through the distal end of an intubation tube in accordance with an embodiment of the invention, showing a detailed view of its soft cuff in an inflated state;
Figure 3B is a cross section through the distal end of an intubation tube in accordance with a further embodiment of the invention, showing a detailed view of its soft cuff in a deflated state;
Figure 3C is a cross section through the intubation tube of Figure 3B, displaying the soft cuff in an inflated state;
Figure 3D is a cross section through an inflatable occluder according to a still further embodiment of the present invention, the occluder comprising two soft cuffs, which are shown in an inflated state;
Figure 3E shows a cross section through an inflatable occluder according to a still further embodiment of the present invention, wherein the coolant gas is used to inflate the occluder;
Figure 3F shows a cross section through an inflatable occluder according to yet another embodiment of the present invention, which is formed integrally with the intubation tube;
Figure 3G shows a cross section through an intubation tube according to yet another embodiment of the present invention, showing the two lumens with the intubation tube;
Figure 3H shows a cross section through an intubation according to a still further embodiment of the present invention, having a different arrangement of lumens to the embodiment of Figure 3G;
Figure 4 shows an inflation bulb, which is used to inflate an inflatable occluder of apparatus according to an embodiment of the present invention;
Figure 5 shows a saline drip attachment, which is used to inflate an inflatable occluder of apparatus according to another embodiment of the present invention;
Figure 6 shows an adapter, which may be used to connect the inflation and cooling gas sources to the intubation tube;
Figures 7A and 7B show the nasal clip of Figure 1 in relaxed and expanded configurations respectively;
Figures 8A and 8B show a nasal clip according to a further embodiment of the present invention in relaxed and expanded configurations.

### Detailed Description

Certain embodiments of the present invention provide a novel, improved form of apparatus for cooling the brain of a patient who is at risk of suffering brain damage as a result of a trauma, such as a stroke. They may provide such apparatus which can be used in a First Aid context by paramedics and other emergency service workers in situations where a person has suffered a trauma, such as a stroke, in the course of his or her normal occupations. In addition, they may provide such apparatus which can be used quickly and safely under emergency situations without risk of causing damage to the lower part of the pharynx.

There will now be described a preferred embodiment of an apparatus for cooling the brain in accordance with the invention, which is illustrated in the accompanying drawings. This embodiment comprises a pernasal intubator comprising a tube having a proximal portion, a lumen extending from the proximal portion, a distal end for insertion through one of the nostrils of a patient requiring brain cooling, an inflatable occluder provided on a distal portion of the tube and in communication with the lumen so as to permit inflation of the inflatable occluder by fluid supplied through the lumen thereby to occlude the nasopharynx of the patient behind the soft palate of the patient and to prevent passage of fluid, i.e. liquid or gas, between the nasal cavities and the mouth of the patient. It further comprises a pressure device for application to the exterior of the nose of the patient so as to apply pressure to the angular vein of the patient thereby to increase venous blood flow to the patient's brain. In addition, it comprises a coolant gas supply for supplying coolant gas to at least one of the nasal cavities of the patient.

Preferably the lumen is provided art its proximal end with a fluid connector for connection to a source or generator of pressurised fluid. Such a fluid connector can be adapted for connection to an inflation bulb, for example.

The lumen can have a closed distal end in which case an aperture or apertures is provided in the wall of the lumen in fluid communication with the inflatable occluder whereby introduction of fluid, either liquid or a gas, through the lumen causes inflation of the inflatable occluder.

The inflatable occluder conveniently comprises a first cuff secured in fluid tight fashion to a distal portion of the tube. Alternatively the inflatable occluder may comprise a balloon secured in fluid tight fashion to a distal portion of the tube. In this case the tube may have an open distal end located within the balloon. If desired, the tube may be provided with a subsidiary inflatable cuff positioned proximally upstream from the first cuff or balloon.

Any non-toxic gas or mixture of gases can be used as coolant gas. Preferably, however, the coolant gas is oxygen. The coolant gas is preferably supplied at a temperature of from about -50° C to about +10° C. It can be supplied, for example, at a rate of from about 1 l/min to about 100 l/min (measured at 0° C and at 1 bar).

The pressure device conveniently comprises a nasal clip. Preferably the pressure device is adapted for securement on the tube.

In addition, there is described a method of cooling the brain of a patient in danger of suffering brain damage which comprises occluding the air passage leading from the nasal cavities to the mouth cavity of the patient behind the soft palate of the patient, applying pressure to an exterior part of the patient's nose so as to exert pressure on the angular vein of the patient thereby to increase venous blood flow to the patient's brain, and supplying a coolant gas to the nasal cavities of the patient. Such a method does not involve obstruction of the oropharynx or laryngopharynx and the patient can continue to breathe through his or her mouth with or without the aid of a respirator.

Conveniently in such a method the coolant gas is supplied through one of the patient's nostrils and discharged through the other nostril. The coolant gas can be supplied intermittently but is preferably supplied continuously.

A particularly preferred method, not being part of the invention, of cooling the brain of a patient suffering from a condition which could lead to brain damage comprises the steps of:
providing a pernasal intubator comprising a tube having a proximal portion, a lumen extending from the proximal portion, a distal end, and inflatable occluder provided on a distal portion in fluid communication with the lumen;
introducing the distal end of the tube through one of the nostrils of the patient until the inflatable occluder lies in the nasopharynx behind the soft palate of the patient;
inflating the inflatable occluder by supplying fluid through the lumen so as to occlude the nasopharynx of the patient in a region behind the soft palate of the patient, thereby to prevent passage of liquid between the nasal cavities and the mouth of the patient;
applying pressure to an exterior part of the patient's nose so as to apply pressure to the angular vein of the patient thereby to increase venous blood flow to the patient's brain;
supplying coolant gas to at least one of the nasal cavities of the patient; and
allowing gas to exit through at least one of the patient's nostrils.

The tube is inserted through the patient's nostril until the inflatable occluder lies behind the patient's soft palate and then the inflatable occluder is inflated so as to block the passage of gas and liquid between the patient's nasal cavities and the patient's mouth. In this way the oropharynx and laryngopharynx of the patient are left substantially unobstructed so that the patient can continue to breathe through the mouth either unaided or with the assistance of a respirator.

Inflation of the inflatable occluder may comprise supplying air under pressure through a second lumen in the pernasal intubation tube, for example, by an inflation bulb.

Coolant gas can be supplied to the patient's nasal cavities through a second tube inserted through the patient's same nostril as the pernasal intubator or, preferably, through the patient's other nostril. Alternatively the tube of the pernasal intubator can be provided with a second lumen for supply of coolant gas terminating at an orifice in the wall of the tube of the pernasal intubator disposed at a position such that, in use, it will lie within one of the patient's nasal cavities.

Conveniently the coolant gas is supplied from a suitable supply, such as a pressurised gas cylinder, and cooled as a result of adiabatic expansion or by passage of the gas through a cooling coil immersed in a cooling bath, such as an acetone/solid CO₂ (dry ice) cooling bath or a refrigerated brine solution.

As already noted, gas is permitted to escape through one or other, or both, of the patient's nostrils. Accordingly it is necessary not to block at least one the patient's nostrils in use of the pernasal intubator.

It will normally be preferred that the temperature of the brain is monitored and that the coolant gas is introduced at a rate sufficient to maintain the temperature of the brain at between about 32°C and about 35 °C.

In the drawings the reference numerals indicate like parts throughout.

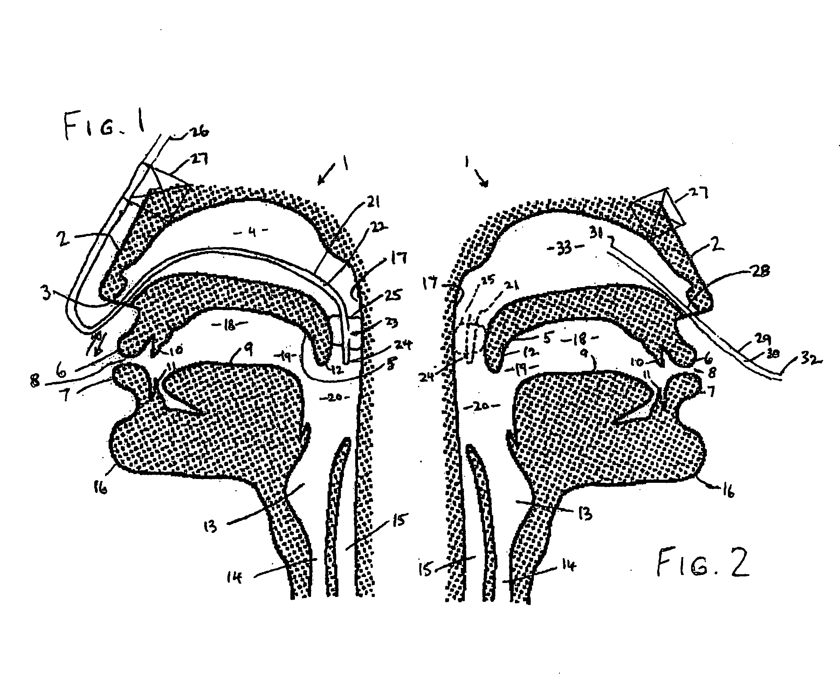

Referring to the drawings, Figure 1 is a semi-diagrammatic cross section through part of a person's head 1, including the nose 2, right nostril 3, right nasal cavity 4, soft palate 5, lips 6 and 7, mouth 8, tongue 9, teeth 10 and 11, epiglottis 12, larynx 13, trachea 14, oesophagus 15, chin 16, nasopharynx 17, mouth cavity 18, oropharynx 19, and laryngopharynx 20.

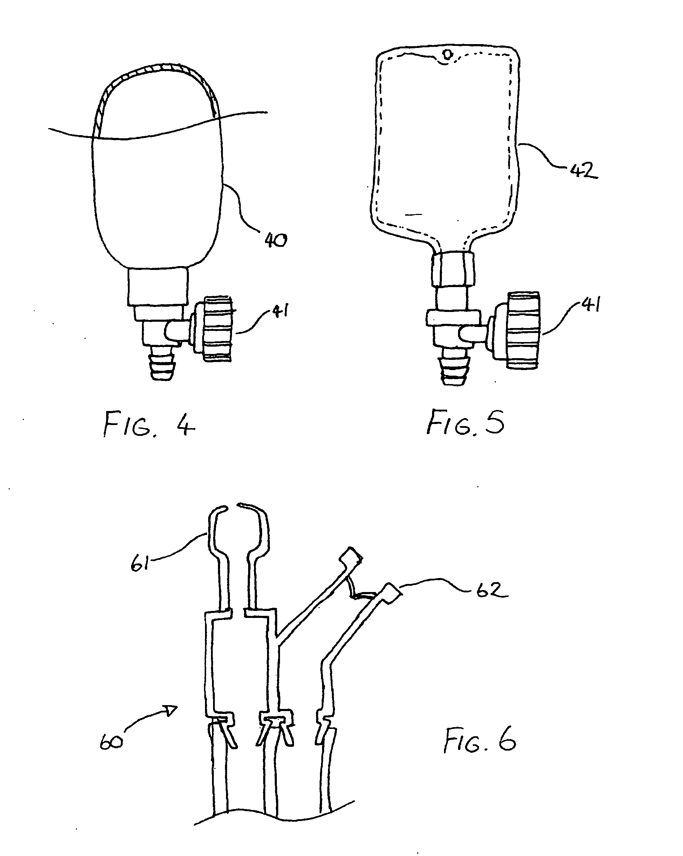

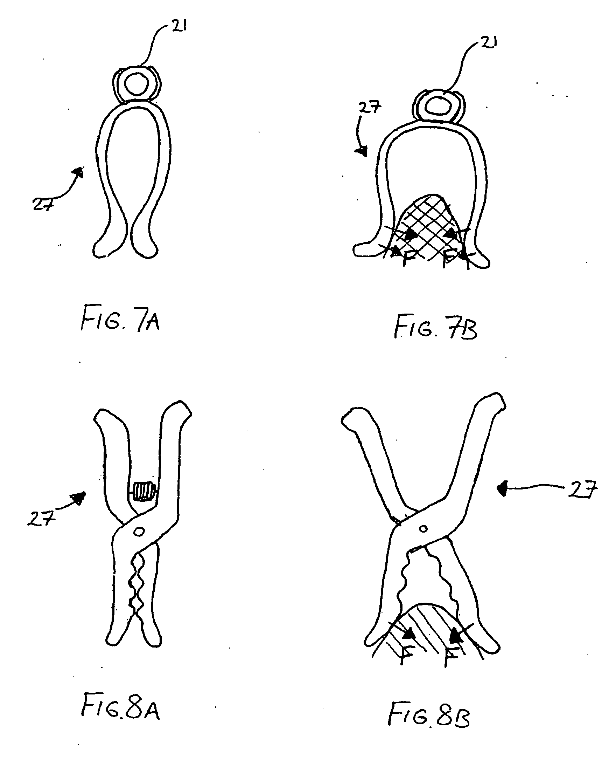

The illustrated apparatus includes a pernasal intubation tube 21 which has a lumen 22 that leads to an aperture 23 near a distal end 24 of tube 21. As illustrated the pernasal intubation tube 21 has been inserted through the patient's right nostril 3; however, it can alternatively be inserted though the patient's left nostril, if more convenient, perhaps as a result of the way that the patient is lying. Tube 21 carries an inflatable cuff 25 near its distal end 24 and aperture 23 lies within cuff 25. Tube 21 further has a proximal end 26 which is provided with an appropriate connection device (such as is shown in Figure 6) in communication with the lumen 22 for connection to a source of an inflation fluid, such as air, oxygen, or of a liquid, such as a cold saline solution. In the embodiment shown in Figure 4 an inflation bulb 40 similar to that used as part of a mechanical sphygmomanometer is connected to the proximal end 26 for supply of air through lumen 22 for inflating the inflatable cuff 25. The inflation bulb 40 is connected to a valve 41, which is opened or close in order to control inflation. Figure 5 shows a cold saline dispenser 42, having a similar valve 41 to the inflation bulb 40 of Figure 4. The saline dispenser may be a flexible polymer bag as shown in the Figure, which may be attached to a line and raised in order to generate sufficient fluid pressure. A clip 27 made of a resilient material is secured to tube 21. In use, clip 27 can be applied, as illustrated, to the external surface of the patient's nose so as to exert pressure forces F (see Figures 7A, 7B, 8A and 8B) on the skin and hence on the underlying angular vein of the patient thereby to increase venous blood flow to the patient's brain.

Figure 2 shows a section through the left side of the patient's face and left nostril 28. A second tube 29 having a lumen 30 leading to an open distal end 31 and connected at its proximal end 32 to a source of coolant gas (not shown), such as cold oxygen, is inserted loosely through right nostril 28 into the right nasal cavity 33. By passing cold coolant gas through second tube 29 into the patient's nasal cavities at an appropriate rate, while pinching off the blood flow through the patient's angular vein by nasal clip 27 so as to increase the flow of venous blood to the patient's brain, the patient's brain can be cooled. Since neither pernasal intubation tube 21 nor the second tube 29 blocks either of the patient's nostrils, air and spent coolant gas, such as oxygen, can escape freely from the patient's nasal cavities.

Although the apparatus has been illustrated in the accompanying semi-diagrammatic drawings with the pernasal intubation tube 21 inserted through the patient's right nostril 3 (as shown in Figure 1) and with the second tube 29 inserted in the patient's left nostril 28 (as shown in Figure 2), it will be readily appreciated by the skilled reader that it does not matter which through which nostril the pernasal intubation tube 21 is inserted. Thus the pernasal intubation tube 21 can alternatively be inserted through the patient's left nostril 28 while the second tube 29 is inserted through the patient's right nostril 3.

In use of the illustrated apparatus for First Aid treatment of a patient in danger of suffering brain damage after a stroke (which is of course only one example of usages to which apparatus according to the present invention may be put), the patient will typically be lying, or made to lie, prone on his or her back or side. Having ensured that the patient is in an appropriate position, a paramedic or other medically qualified practitioner then inserts the pernasal intubation tube 21 through one of the patient's nostrils, for example, through the patient's left nostril 3, until its closed distal end 24 lies just below the patient's soft palate 5. The paramedic can then check visually through the patient's mouth 8 to confirm that the distal end 24 is in the correct position. Next the inflatable cuff 25 is inflated using, for example, an inflation bulb until inflatable cuff 25 occludes the nasopharynx 17. It will be noted that the oropharynx 19 remains substantially unobstructed so that the patient can continue to breathe through his or her mouth 8 either unaided or with the help of a respirator (not shown).

A typical inflation pressure for inflating cuff 25 is about 100 mb gauge (about 1.45 psig).

The second tube 29 is inserted in the patient's other nostril, for example, the left nostril 28.

Temperature sensors can be positioned in the patient's auditory canals or attached to the patient's neck or other areas of the head or body in order to monitor the patient's brain temperature. In addition the body temperature of the patient can be monitored with, for example, the aid of a rectal thermometer.

In a First Aid situation, once the pernasal intubation tube 21 and the second tube 29 are in place, the coolant gas supply can be turned on so as to cause coolant gas, for example, cold oxygen, to pass through second tube 29 into the patient's nasal cavities thereby cooling directly the lining of the nasal cavities 4 and 33 and also the blood in the subjacent arteries and veins.

It will be appreciated by those skilled in the art that the illustrated apparatus is simple and readily portable. It is thus well suited for use by paramedics or by persons with appropriate training in first aid, such as police officers, who are likely to be first on the scene when a person suffers a stroke in the course of his or her usual occupations. Insertion of the pernasal intubation tube 21 through one of the patient's nostrils 3 or 28 can be achieved quickly and simply without the need for any additional probe or special intubation device. Moreover it can readily be checked visually whether the distal end 24 is in the correct position behind the patient's soft palate 5 prior to inflation of cuff 25. Similarly insertion of the second tube 29 in the patient's other nostril 28 or 3 is likewise quick and simple.

In comparison with prior art techniques that involve lowering the temperature of the entire body, the various forms of apparatus and the methods described herein have the advantage that only localised lowering of temperature of the head region is effected in order to cause cooling of the brain.

If desired, an electric pump designed to produce and maintain a predetermined pressure may be used in place of an inflation bulb for the purpose of inflating cuff 25.

Any non-toxic gas or mixture of gases can be used as coolant gas for supply through tube 29. Preferably, however, the coolant gas is oxygen. The coolant gas is preferably supplied at a temperature of from about -50° C to about +10° C. It can be supplied, for example, at a rate ranging from about 1 l/min to about 100 l/min (measured at 0° C and at 1 bar). It can be supplied directly from a pressurised oxygen cylinder, the desired lowered temperature being achieved by virtue of adiabatic expansion of the oxygen as it emerges from the cylinder. If a lower temperature is desired, then the coolant gas can be passed through a coolant coil immersed in a cooling bath containing, for example, refrigerated brine or an acetone/solid CO₂ (dry ice) mixture. Alternatively the coolant gas can comprise air which has been bubbled through liquid oxygen.

In the course of treatment of a patient to effect brain cooling, it may be desirable that the patient's brain temperature is reduced to a temperature of not less than about +30° C, preferably not less than about +32° C, e.g. about +32° C to about +35° C. Such reduced brain temperature can be maintained, for example, for a period of a few hours up to several few days or even months. A typical period of treatment may involve maintaining such a reduced brain temperature for from about 24 hours to about 72 hours. After receiving First Aid to reduce the brain temperature in the event of a stroke or other trauma that could lead to brain damage, the patient can undergo further suitable treatment in a hospital environment to lower his or her body temperature, in addition to reducing his or her brain temperature by the described method.

The materials of construction of the apparatus of the invention can be selected from among those conventionally used in the field of medical intubation devices. For example, pernasal intubation tube 21 can be made from a pliable semi-rigid, soft plastics material such as a medical grade of polyethylene, polypropylene, polyvinylchloride, or the like. If desired, the distal end of pernasal intubation tube 21 may be coloured, e.g. blue or green, so as to increase its visibility and make it easier for a paramedic or other appropriately trained person to check that it is correctly in position before cuff 25 is inflated. The distal tip 24 may also include a radiopaque material to facilitate its visualisation in X-ray photographs.

Inflatable cuff 25 can be made from any material conventionally used for manufacture of cuffs on intubation devices, such as a medical grade of a silicone rubber.

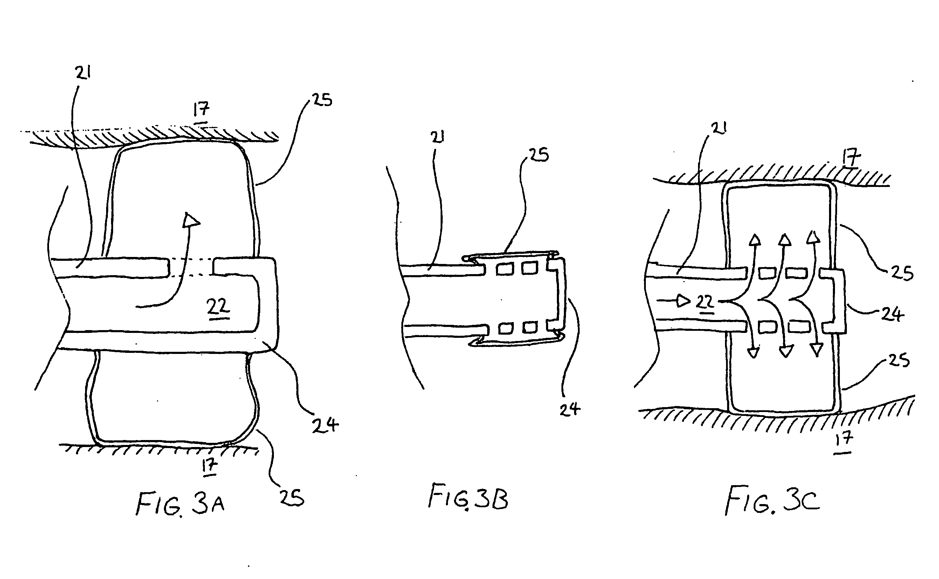

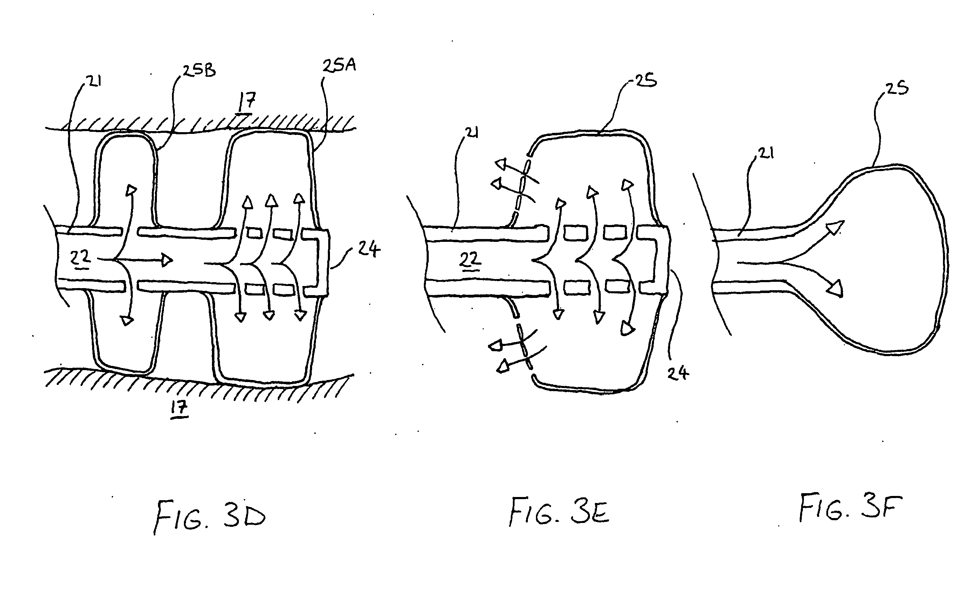

Figure 3A shows in more detail the pernasal intubation tube 21 of Figures 1 and 2, having a single inflatable cuff 25 adjacent its distal end 24 which is sealed or otherwise secured in airtight fashion to the tube 21 both proximally and distally with respect to the aperture 26. Figures 3B and 3C show in deflated and inflated states respectively an alternative embodiment in which the tube 21 has several apertures to allow passage of the inflation fluid into the cuff 25. Alternatively, as is shown in Figure 3F cuff 25 can be replaced by a balloon positioned at the distal end 24, being sealed or secured thereto in liquid tight fashion by a single line of sealing or the like proximally adjacent the distal end 24; in this case lumen 22 can lead to an open distal end 24 and aperture 23 is not then required. In the particular example shown in Figure 3F, the thickness of the tube tapers so that its distal end may be inflated, thus providing an integrally formed balloon 25.

As shown in Figure 3D, it is also envisaged that a second cuff 25B can be provided on pernasal intubation tube 21 to assist in occluding the nasopharynx, this second cuff 25B being positioned proximally with respect to cuff 25 and the lumen 22 having a further aperture or apertures for inflation of the second cuff. Alternatively the pernasal intubation tube 21 can be provided with a second lumen for inflating such a second cuff.

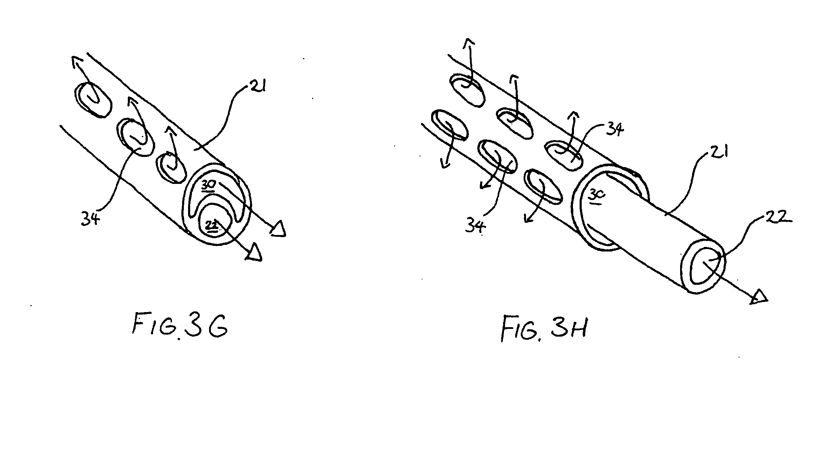

As described with reference to Figures 1 and 2, separate lines or tubes may be used to inflate the inflatable occluder and to supply cooling gas into the nasal passages. However, it is also envisaged that these two tubes may in some constructions be combined as separate lumens of a single tube. Figures 3G and 3H show embodiments of the present invention having two such lumens: an inflation fluid carrying lumen 22 and a coolant carrying lumen 30. The lumens may be concentric as in Figure 3H, but may equally be separate and parallel as in Figure 3G. Apertures 34 are formed in the wall of the tube enclosing lumen 30 to allow egress of coolant fluid.

It is also envisaged that, in certain embodiments, the coolant gas may be used to inflate the cuff 25 of the apparatus. In such constructions a single lumen tube may be used, with apertures formed in the wall of the tube. In the particular embodiment shown in Figure 3E, apertures are formed within the proximal surface of the inflatable cuff 25 and in the distal end of the tube 21. Thus, the inflatable cuff is inflated by the coolant gas, a portion of which is released backwards into the nasal passages to cool the brain.

As described, cuff 25 is arranged to be inflated with air under pressure. However, it is also envisaged that it can alternatively be inflated using liquid under pressure. Hence it can be arranged for connection via an inlet tap or valve to a diffusion bag filled, for example, with a cold saline solution, the appropriate pressure being generated by elevating the bag to an appropriate height above the patient's head.

A connector such as that depicted in Figure 6 may be used for connection of the source of inflation fluid to the tube 21. This connector also includes both an adapter for the inflation fluid source 61 and a side port adapter 62 for connection of the coolant gas supply.

In place of the wishbone shaped clip of Figures 1, 2, 7A and 7B there may alternatively be used a hinged spring-loaded clip with two legs, opposed adjacent ends of which are urged towards each other by a spring, an exemplary construction being shown in Figures 8A and 8B, which is similar to that of a conventional spring-loaded laundry peg.

## Claims

1. Apparatus for cooling the brain comprising:
a pernasal intubation tube having a proximal portion, a lumen extending from the proximal portion, a distal end for insertion through one of the nostrils of a patient requiring brain cooling,
an inflatable occluder provided on a distal portion of the tube and in communication with the lumen for inflation by fluid supplied through the lumen thereby to occlude the nasopharynx of the patient behind the soft palate of the patient and to prevent passage of fluid between the nasal cavities and the mouth of the patient;
a pressure device for application to the exterior of the nose of the patient so as to apply pressure to the angular vein of the patient thereby to increase venous blood flow to the patient's brain; and
a coolant gas supply for supplying coolant gas to at least one of the nasal cavities of the patient.

2. Apparatus according to claim 1 wherein the inflatable occluder comprises a first cuff secured in fluid tight fashion to a distal portion of the tube.

3. Apparatus according to claim 2 wherein the tube is provided with a subsidiary inflatable cuff positioned proximally upstream from the first cuff.

4. Apparatus according to claim 1 wherein the inflatable occluder comprises a balloon secured in fluid tight fashion to a distal portion of the tube.

5. Apparatus according to claim 4 wherein the tube is provided with a subsidiary inflatable cuff positioned proximally upstream from the balloon.

6. Apparatus according to any one of claims 1 to 5 wherein a fluid connector is provided at the proximal end of the lumen for connection to a source or generator of pressurised fluid.

7. Apparatus according to claim 6 wherein the fluid connector is adapted for connection to an inflation bulb.

8. Apparatus according to any one of claims 1 to 7 wherein the coolant gas supply comprises a second tube for insertion in one of the patient's nostrils.

9. Apparatus according to any one of claims 1 to 8 wherein the pressure device comprises a clip for releasable securement on the patient's nose.

10. Apparatus according to claim 9 wherein the coolant gas supply comprises a second tube for insertion in one of the patient's nostrils and wherein the clip is adapted for securement to the second tube.

## Patentansprüche

1. Vorrichtung zur Kühlung des Gehirns, umfassend:
einen pernasalen Intubationsschlauch mit einem proximalen Abschnitt, einem sich von dem proximalen Abschnitt erstreckenden Lumen, einem distalen Ende zur Einführung durch eines der Nasenlöcher eines Patienten, der eine Gehirnkühlung benötigt,
einen aufblasbarer Okkluder, der auf einem distalen Abschnitt des Schlauch vorgesehen ist und mit dem Lumen in Verbindung steht, damit er durch Flüssigkeit, die durch das Lumen zugeführt wird, aufgeblasen werden kann und so den Nasenrachenraum des Patienten hinter dem weichen Gaumen des Patienten verschließt und den Durchtritt von Flüssigkeit zwischen den Nasenhöhlen und dem Mund des Patienten verhindert;
eine Druckvorrichtung zur Applikation auf die Außenseite der Nase des Patienten zum Aufbringen von Druck auf die Vena angularis des Patienten und so zur Erhöhung des venösen Blutflusses zum Gehirn des Patienten; und
eine Kühlgaszuführung zum Zuführen von Kühlgas zu zumindest einer der Nasenhöhlen des Patienten.

2. Vorrichtung nach Anspruch 1, worin der aufblasbare Okkluder eine erste Manschette umfasst, die flüssigkeitsdicht an einem distalen Abschnitt des Schlauchs befestigt ist.

3. Vorrichtung nach Anspruch 2, worin der Schlauch mit einer aufblasbaren Nebenmanschette versehen ist, die proximal stromaufwärts von der ersten Manschette angeordnet ist.

4. Vorrichtung nach Anspruch 1, worin der aufblasbare Okkluder einen Ballon umfasst, der flüssigkeitsdicht an einem distalen Abschnitt des Schlauchs befestigt ist.

5. Vorrichtung nach Anspruch 4, worin der Schlauch mit einer aufblasbaren Nebenmanschette versehen ist, die proximal stromaufwärts vom Ballon angeordnet ist.

6. Vorrichtung nach einem der Ansprüche 1 bis 5, worin ein Flüssigkeitsverbinder am proximalen Ende des Lumens zur Verbindung mit einer Druckflüssigkeitsquelle oder einem Druckflüssigkeitsgenerator vorgesehen ist.

7. Vorrichtung nach Anspruch 6, worin der Flüssigkeitsverbinder zur Verbindung mit einem Gebläseball ausgelegt ist.

8. Vorrichtung nach einem der Ansprüche 1 bis 7, worin die Kühlgaszuführung einen zweiten Schlauch zur Einführung in eines der Nasenlöcher des Patienten umfasst.

9. Vorrichtung nach einem der Ansprüche 1 bis 8, worin die Druckvorrichtung eine Klemme zur lösbaren Befestigung an der Nase des Patienten umfasst.

10. Vorrichtung nach Anspruch 9, worin die Kühlgaszuführung einen zweiten Schlauch zur Einführung in eines der Nasenlöcher des Patienten umfasst und worin die Klemme zur Befestigung an dem zweiten Schlauch ausgelegt ist.

## Revendications

1. Appareil pour refroidir le cerveau, comprenant:
un tube d'intubation pernasale présentant une partie proximale, une lumière s'étendant à partir de la partie proximale, et une extrémité distale à insérer à travers une des narines d'un patient nécessitant un refroidissement du cerveau;
un obturateur gonflable prévu sur une partie distale du tube et en communication avec la lumière à gonfler par un fluide fourni à travers la lumière, obturant de ce fait le pharynx nasal du patient derrière le voile du palais du patient et empêchant le passage de fluide entre les cavités nasales et la bouche du patient,
un dispositif de pression à appliquer sur l'extérieur du nez du patient de manière à exercer une pression sur la veine angulaire du patient, augmentant de ce fait le débit sanguin veineux vers le cerveau du patient; et
une alimentation en gaz de refroidissement pour fournir un gaz de refroidissement à au moins une des cavités nasales du patient.

2. Appareil selon la revendication 1, dans lequel l'obturateur gonflable comprend un premier parement fixé d'une façon étanche au fluide à une partie distale du tube.

3. Appareil selon la revendication 2, dans lequel le tube est pourvu d'un parement gonflable subsidiaire qui est positionné de façon proximale en amont du premier parement.

4. Appareil selon la revendication 1, dans lequel l'obturateur gonflable comprend un ballon qui est fixé d'une façon étanche au fluide à une partie distale du tube.

5. Appareil selon la revendication 4, dans lequel le tube est pourvu d'un parement gonflable subsidiaire qui est positionné de façon proximale en amont du ballon.

6. Appareil selon l'une quelconque des revendications 1 à 5, dans lequel un connecteur de fluide est prévu à l'extrémité proximale de la lumière afin d'être connecté à une source ou à un générateur de fluide sous pression.

7. Appareil selon la revendication 6, dans lequel le connecteur de fluide est adapté pour être connecté à une poire de gonflage.

8. Appareil selon l'une quelconque des revendications 1 à 7, dans lequel l'alimentation en gaz de refroidissement comprend un deuxième tube à insérer dans l'une des narines du patient.

9. Appareil selon l'une quelconque des revendications 1 à 8, dans lequel le dispositif de pression comprend une pince à fixer de façon détachable sur le nez du patient.

10. Appareil selon la revendication 9, dans lequel l'alimentation en gaz de refroidissement comprend un deuxième tube à insérer dans l'une des narines du patient, et dans lequel la pince est adaptée pour être fixée au deuxième tube.
